# EUROPEAN PATENT SPECIFICATION

(11) **EP 0 647 199 B1**
(45) Date of publication and mention of the grant of the patent: **03.05.2000**
(21) Application number: 93915429.0
(22) Date of filing: 16.06.1993
(51) Int. Cl.: B65D 75/00, B65D 71/00

(54) **A PACKAGE FOR PACKS, FOR EXAMPLE, PACKS OF SANITARY PRODUCTS**
VERPACKUNG FÜR VERPACKUNGEN, Z.B. PACKUNGEN FÜR HYGIENEARTIKEL
EMBALLAGE POUR PAQUETS TELS QUE DES PAQUETS DE PRODUITS HYGIENIQUES

(30) Priority: 19.06.1992 IT TO920526
(43) Date of publication of application: 12.04.1995
(73) Proprietor: FATER S.P.A., I-65128 Pescara (IT); THE PROCTER & GAMBLE COMPANY, Cincinnati, Ohio 45202 (US)
(72) Inventor: MARINELLI, Luigi, I-65122 Pescara (IT); PALUMBO, Gianfranco, I-65125 Pescara (IT)
(74) Representative: Bottema, Johan Jan
(86) International application number: US9305897
(87) International publication number: WO9400362

(56) References cited:
- CH-A- 394 944
- DE-A- 4 120 480
- DE-A- 4 213 463
- US-A- 1 764 569
- US-A- 2 062 539
- US-A- 2 666 710
- US-A- 2 703 764
- US-A- 3 246 744
- US-A- 4 079 566
- US-A- 4 669 611

## Description

The present invention relates to a package, particularly but not exclusively for packs of sanitary products, for example, plastics packs for products such as babies' disposable nappies.

Plastics bags are becoming the most widespread type of pack for sanitary products such as babies' disposable nappies and are particularly suitable for compressed products, that is, products which have been subjected to a certain degree of compression before insertion in the bags in order to obtain smaller packs which occupy less space and use less raw material.

Once packed, the bags are packaged in corrugated cardboard boxes for despatch; the cardboard box simplifies the handling and transportation of the bags and also constitutes a sales unit for the product.

However, cardboard boxes represent a considerable quantity of material which has to be purchased and stored by the user who uses it to pack products and which, once the goods have arrived at their destination, is then generally disposed of by the purchaser.

Moreover, when bags of disposable nappies are packaged in cardboard boxes the space inside the boxes cannot be fully utilised because of the tolerances imposed by the usual automatic mechanical boxing systems generally used.

The problem of the utilization of space is made even more important by the increasingly widespread use of pallets of standard dimensions for the transportation of the boxes, in response to the requirements of large distribution and sales organisations.

US 2,703,764 discloses a package as described in the preamble of appended claim 1. In this package a number of units are assembled together in pairs and in a stacked relationship. Band tapes serve to maintain all the pairs as a rigid unit. To remove one of the pairs from the unit, it is only necessary to twist such a pair in either direction to cause the tape to tear at the meeting edges of the pairs to be separated. The tape comprises embrittled portions to facilitate the twisting. Furthermore, spots of adhesive may be provided at the meeting edges of one or more of the respective pairs in order to anchor the respective pairs more effectively.

The object of the present invention is to improve the characteristics of packages, for example, for plastics packs of sanitary hygiene products, such as babies' disposable nappies, by means of a package which avoids the use of corrugated cardboard boxes and which has the characteristics recited in the following claims.

Further characteristics and advantages of the invention will become clear from the following description, given purely by way of non-limiting example with reference to the appended drawings, in which:
Figure 1 is a perspective view of a conventional package of four plastics packs of sanitary hygiene products which are housed in a corrugated cardboard box with the box shown transparently to show its contents more clearly,
Figure 2 is a perspective view of the same four packs, packaged according to the present invention,
Figure 3 shows a portion of the adhesive tape used to form the package according to the present invention,
Figure 4 is a view of a roll of adhesive tape from which the portion shown in Figure 3 can be formed,
Figures 5-9 are perspective views of a corresponding number of alternative configurations of a package of four packs formed according to the present invention,
Figure 10 is a perspective view of the arrangement of corrugated boxes of the type illustrated in figure 1 on a pallet,
Figure 11 is a perspective view of the arrangement of packages formed according to the present invention on a pallet of the same type, and
Figures 12-19 show a perspective schematic view of different configurations of a package according to the invention.

The package of the present invention will be described herein, by way of example, in relation to its use for plastics packs of sanitary products such as babies' disposable nappies.

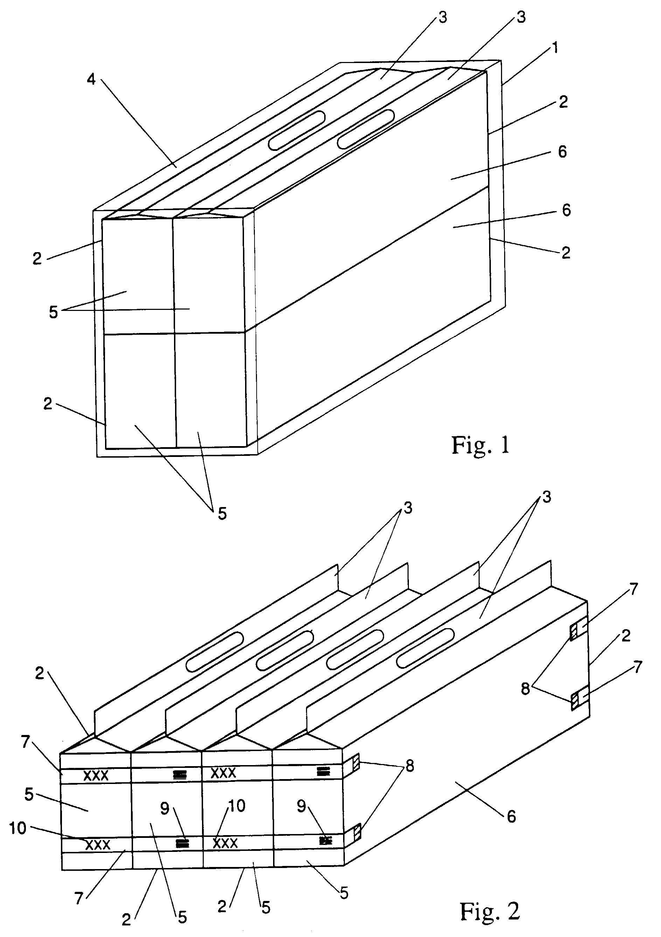

By way of reference, Figure 1 shows a conventional package constituted by a corrugated cardboard box 1 containing four plastic packs 2 of disposable nappies.

The packs 2 are of the type commonly available commercially and generally comprise a handle 3 at the top to enable the user to carry them.

Figure 1 shows one of the possible configurations with the four packs 2 disposed in the two superposed layers within the box 1.

To enable the packs 2 to be inserted by packaging machines, however, some of the space in the box is not filled; Figure 1 shows the spaces 4 left between the packs 2 and the walls of the box 1 along its longer sides.

Figure 2 shows the same packs 2 packaged according to the present invention; the packs are placed one beside the other in a single row with their longer sides next to each other in a manner such that their eight shorter sides 5, four adjacent sides at each end, the two outer longer sides 6 of the two end packs and, finally, the upper and lower faces of the packs, face outwardly.

In the configuration shown, the packs are bound together by means of at least four pieces of adhesive tape 7 applied, two on each side and parallel to each other, to the two shorter sides 5 of each pack, forming a package which takes up less space than the cardboard box 1 although it contains the same number of packs 2.

The package can easily be picked up and carried by hand by being gripped, for example, by the handles 3 of the two end packs; the handle 3 of each individual pack 2 can preferably support the weight of the entire package.
On each of the two ends of the package formed by the four adjacent shorter sides 5 of the packs 2, one of the two pieces of adhesive tape 7 is positioned high up, a short distance from the upper edges of the shorter sides 5 and parallel thereto, and the other piece is positioned low down, a short distance from the lower edges of the sides 5.

In general, at least one piece of adhesive tape 7 is positioned within the upper halves of the adjacent shorter sides 5 on each side of the package and at least one further piece is positioned within their lower halves; the portion of each shorter side 5 which is between the upper tape and the lower tape is preferably tall enough to include an opening system for the pack of nappies such as, for example, that described in patent application IT 67217 A/90.

In any case the widths of the pieces of adhesive tape 7 should be such that the tapes cover in total at least 20% of the surfaces of the shorter sides 5 of the packs 2.

The two ends of each piece of adhesive tape 7 also extend partially along two outer longer sides 6 in order to improve the grip of the tape and hence the stability of the group of packs.

Each end of each piece 7 has an adhesive-free region 8 which can easily be gripped in order to start the removal of the tape and thus separate one or more packs 2 from the group.

The adhesive tape of which the pieces 7 are formed must have certain characteristics in relation to the substrate constituting the wrappers of the packs of nappies to which it is to be applied, which is typically of printed plastics, for example, polyethylene film.

In particular, the tape should not have a tendency to leave some of the adhesive on the substrate to which it is applied during the removal of the tape from the wrapper of the pack and should not give rise to relative slippage between the tape and the substrate during the life of the package; these characteristics may be expressed in terms of the creep strength of the adhesive tape in relation to the substrate.
The degree of tackiness of the adhesive tape, which can be measured as the peel strength of the adhesive tape in relation to the substrate, should be such as to confer good stability to the group of packs under normal handling and transportation stresses but, at the same time, must enable the tapes to be removed easily when the individual packs are separated from the package; in any case, the peel strength of the adhesive tape should be less than the strength of the substrate to which it is applied so that, when the adhesive tape is removed, the substrate which is made, for example, of the polyethylene film typical of nappy packs, is not torn.

Moreover, the tape must have good long-term stability to ensure constant peel strength and shear strength throughout the life of the package, which includes the periods of time during which it is transported and stored both by the producer and by the customer.

Finally, the tape should have a tensile strength such that it can withstand the stresses to which it may be subjected during the life of the package without breaking

The shear strength was measured by the PSTC7 Test, Method A for measurement at ambient temperature and Method C for measurement at 50°C, which are described in the Ninth Edition of "Test Methods for Pressure Sensitive Tapes" published by the Pressure Sensitive Tape Council, Suite 201, 104 Wilmot Road, Deerfield, IL, U.S.A., and modified as follows.

For both the methods a steel support plate was used, and was covered by a layer made of the same material as the packs 2, typically a polyethylene film 80 microns thick, which had the same dimensions as the plate and was fixed thereto.

The roller used to press the test sample onto the substrate weighed 2 kg and the weight used for the test also weighed 2 kg.

The sample of adhesive tape used for the test was 25.4 mm (1 inch) wide and was stuck to the substrate so as to cover an area of 25.4 x 25.4 mm² (1 square inch).
The test evaluated the ability of an adhesive tape to remain adhering to the substrate under a load applied parallel to the surface of the tape.

One end of the adhesive tape was fixed to the test surface which was disposed vertically, and a weight was applied to the other end; the time required to remove the adhesive tape completely from the test surface under the load exerted by the weight was measured.

The peel strength was evaluated by the "FINAT Test Method No. 2" (FTM2) described in "FINAT Pressure Sensitive Laminates Suppliers and Users Technical Manual", 1985 edition, published by FINAT Pressure Sensitive Technical Committee and available from FINAT Secretariat, Laan Copes Van Cattenburch 79, 2585 EZ, The Hague, NL and modified as follows.

The glass support plate used for the test was replaced by a 50 x 160 mm plate 6 mm thick formed by two 3mm wood fibre panels (faesite) covered on one side with a layer of plastics laminate with a smooth, opaque outer finish, the plate as a whole showed the laminate on both faces.

A layer, having the same dimensions as the plate and made of the material of which the packs 2 are formed, typically a polyethylene film 80 microns thick, was fixed to one face of the plate.

The peel strength of the adhesive tape was tested on the surface of the sheet material fixed to the plate.

The force required to remove and adhesive tape previously applied to a test surface was measured, with an angle of 90° between the direction of the force and the surface.

An adhesive tape from which to form the pieces 7 having the desired characteristics may be constituted by a substrate film of polypropylene 35 microns thick suitably rendered adhesive so that it has a peel strength of between 0.2 N/cm and 2.5 N/cm, preferably between 0.8 N/cm and 1.6 N/cm, and a shear strength of at least 500 min measured at ambient temperature and at least 50-60 minutes measured at 50°C, in relation to the substrate to which it is stuck.

The adhesive tape from which the pieces 7 are formed may be transparent so that, once applied to the packs 2 it does not conceal the surfaces of the packs, which are generally printed.

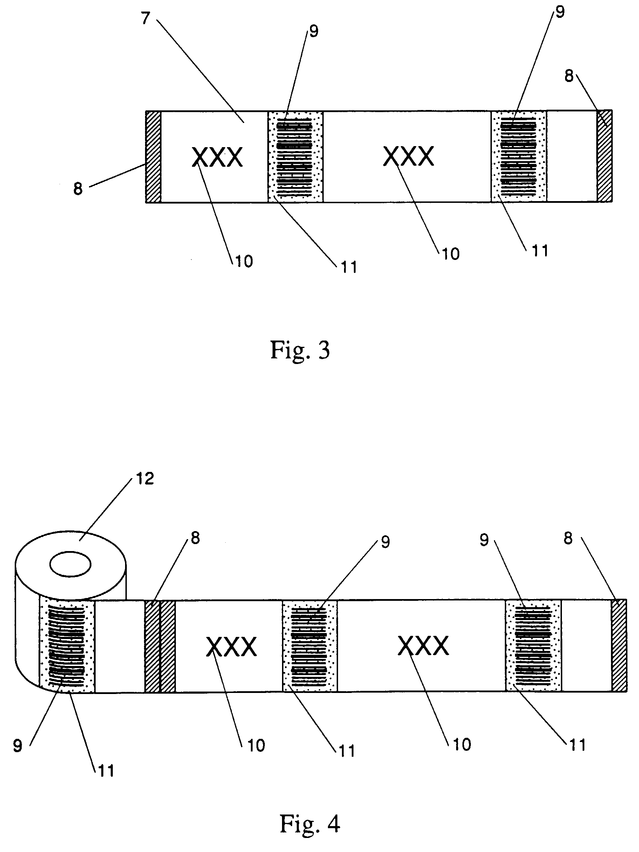

It can be seen from Figure 3 that a bar code 9 for the automatic identification of the product during handling and an alphanumeric code 10 for immediate visual identification can preferably be applied to each piece of adhesive tape 7; the codes can be used advantageously both by the manufacturer and by the customer.

In order to prevent the automatic reading of the bar code from being made difficult by the underlying printing on the pack, the portion 11 of adhesive tape corresponding to the bar code may be made opaque, for example, it may have a black background.

Each piece of adhesive tape 7 having the preferred characteristics and also including the adhesive-free regions 8 and the codes 9 and 10 may be formed from a continuous tape wound in the form of a roll 12, as shown in Figure 4.

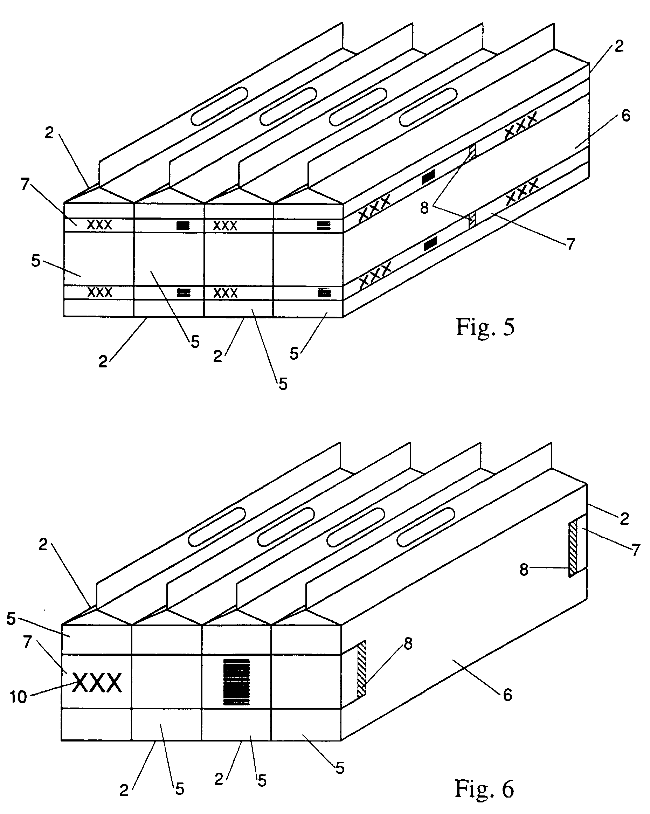

Figures 5-9 show some alternative configurations of the package formed according to the present invention; the numerals used in these drawings refer to the same elements as in Figure 2.

Figure 5 shows a package similar to that of Figure 2 with two pieces of adhesive tape 7 wound all the way around the shorter sides 5 and the longer sides 6 of the packs 2 and positioned in a similar manner to the four pieces of the configuration shown in Figure 2.

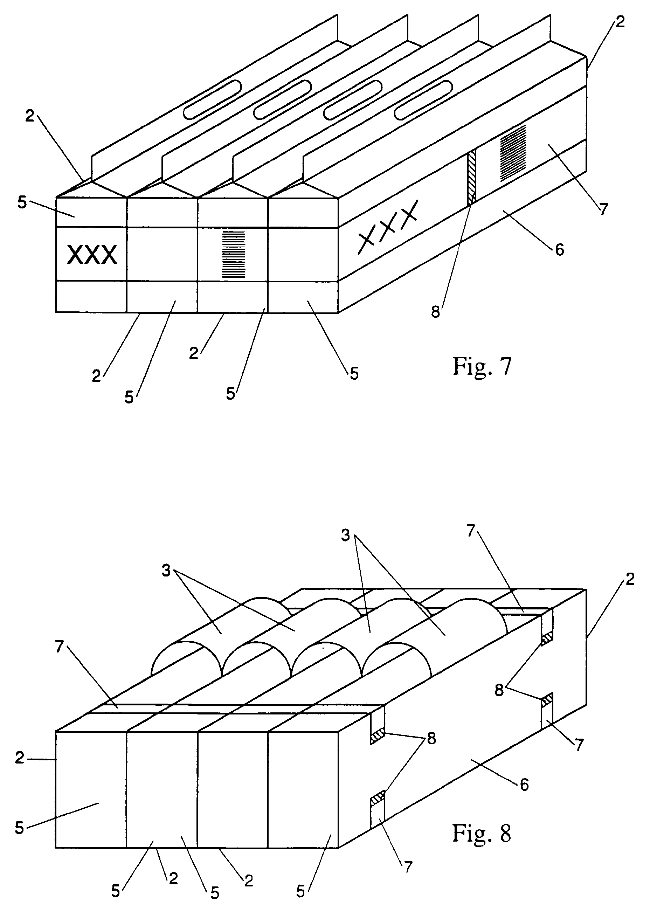

Figures 6 and 7 show a further two alternative configurations of the package of the present invention; in figure 6, the shorter sides 5 of the four packs are bound together by a single piece of adhesive tape 7 at each end, the tape also extending partially on the longer sides 6 and, in the configuration of Figure 7, a single piece of adhesive tape 7 is wound all the way around the four packs 2 on their shorter sides 5 and on their longer outer sides 6. In both cases each piece of adhesive tape 7 is positioned in the center of the adjacent short sides 5, having such a height as to cover preferably at lease 40% of the total surface area of the shorter sides 5 of the packs 8.

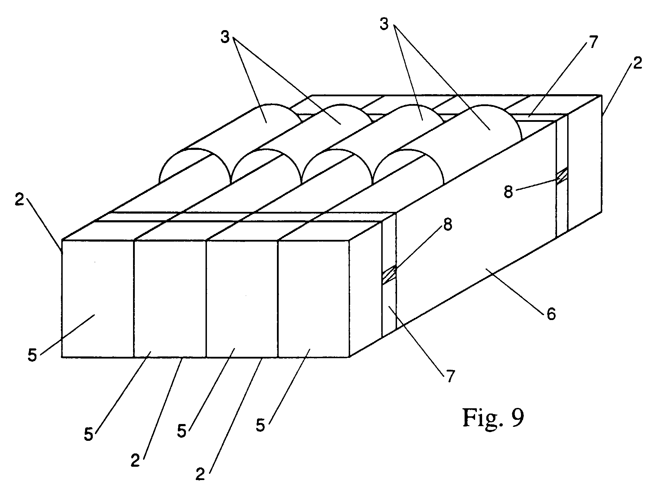

In Figures 8 and 9, the upper and lower faces of the four packs 2 are bound together in a configuration which is particularly suitable for packs 2 each having a handle 3 with dimensions such that it extends solely on the central portion of the upper face, for example, of the type which is applied to the pack rather than being formed integrally therewith, as shown in the drawings.

Figure 8 shows the four packs 2 joined together by two pieces of adhesive tape 7 fixed to the upper faces and by a further two fixed to the lower faces, the pieces also extending partially over the outer longer sides 6.

The pieces of adhesive tape 7 are generally positioned within the third of each upper and lower face which is adjacent the respective shorter side 5, in any case without interfering with the handles 3 disposed on the top; in particular, as shown in Figures 8 and 9, the pieces of adhesive tape 7 are offset towards the shorter edges of the upper and lower faces and are parallel thereto.

In all the alternative configurations shown in Figures 5-9, each end of each piece 7 has an adhesive-free region 8 which can be gripped in order to start the removal of the tape and thus to separate one or more packs 2 from the group. If the piece or pieces of adhesive tape 7 are wound all the way around the packs 2, one end of each piece 7 may be superposed on the opposite end, as shown, in particular, in Figures 5, 7 and 9 which show a single adhesive-free region 8 for each piece 7, on one of the two outer longer sides 6; alternatively, the two ends may be spaced apart and thus both be visible when the package is still intact.

The package according to the present invention can, to advantage, be used with pallets of standard dimensions, the use of which is becoming increasingly widespread at the request of the large distribution and sales organisations.

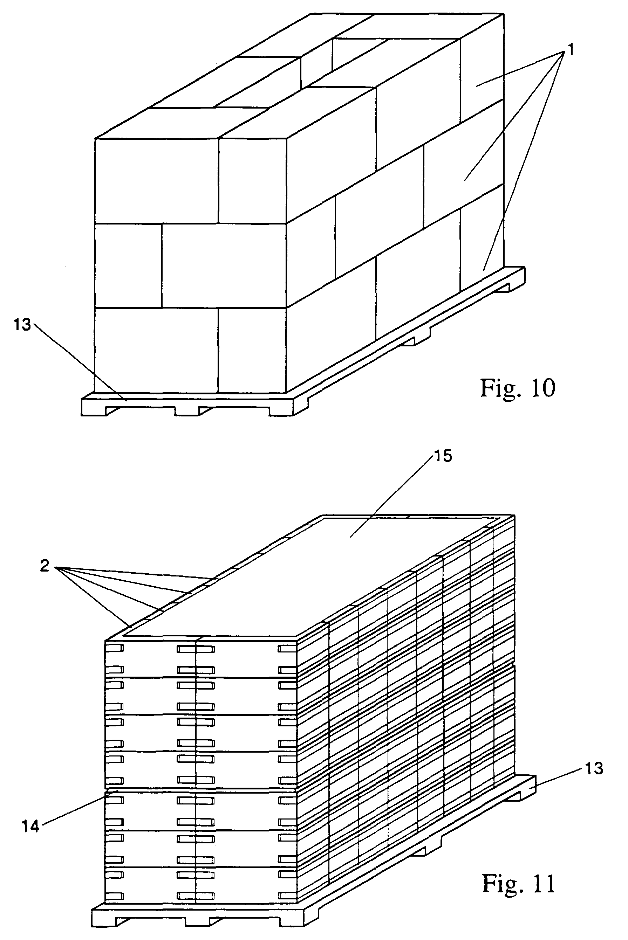

Figures 10 and 11 show two different loading configurations on a pallet 13 of the same type with standard dimensions, for conventional packages with corrugated cardboard boxes such as that shown in Figure 1 and for packages according to the present invention, such as that shown in Figure 2, respectively.

The packages according to the present invention are disposed on the pallet 13 in superposed layers with a sheet of corrugated cardboard 14 disposed between two layers approximately half-way up the whole stack in order to stabilise the stack; a second sheet 15 is preferably added on top of the last layer.

In both cases, the load may be surrounded by a sheet of extensible plastics of the type commonly used for covering and protecting loads on pallets.

By avoiding the use of cardboard boxes, the package according to the present invention eliminates all the costs connected with the provision and use of such boxes and thus represents an advantage both for the manufacturer and for the customer who no longer has to open and empty the boxes in order to make the products accessible or finally to dispose of the empty boxes; the saving of space also achieves and overall reduction in storage and distribution costs.

The new package itself constitutes a sales unit for the product and, moreover, by virtue of the handles 3 of the individual packs 2 and the fact that the product can be recognized more readily than with conventional cardboard boxes, it can be dealt with more easily during the manual handling which is usually carried at the customers premises.

Moreover, the packages can also be positioned on sales shelves as they are without the need to separate the individual packs, the purchaser thus being left to take out the individual packs 2 directly by removing the piece or pieces of adhesive tape 7 which keep it bound to the package, thus being able to separate a single pack 2 at a time, leaving the rest of the package intact.

The products may also be taken to the point of sale directly on the pallets, once the extensible plastics covering sheet has been removed.

In the embodiments illustrated, the flexible plastics packs have dimensions of 145 x 420 x 240 mm (width x length x height) and each contains 36 disposable elasticated nappies of a type commonly available on the market which, in the extended configuration, measure 535 x 350 mm.

The packs are housed in corrugated cardboard boxes with dimensions of 340 x 420 x 520 mm and in packages according to the present invention with dimensions of 580 x 420 x 240 mm, each bound together by four pieces of adhesive tape 800 mm long and 60 mm wide; the pallets have standard dimensions of 1200 x 800 mm.

In the first case there are eighteen cardboard boxes on the pallet in three layers of six with a total height of 1560 mm, leaving an unused empty space in the centre shown by shading in Figure 10; in the second case, there are twenty-eight packages formed according to the present invention, arranged on the pallet in seven layers of four, with a total height of 1700 mm, which is slightly greater than in the first case but within the size limits fixed conventionally for loads on pallets; the saving of space thus achieved is about 50%.

Naturally this saving relates to the configuration of the embodiment illustrated.

In any case, although the dimensions of the individual packs 2 vary, for example, according to the different measurements of nappies and the number of packs which make up the package of the present invention, there will always be a saving of space, greater or less than that shown in the example, in comparison with corresponding conventional packages in cardboard boxes.

Naturally, the principle of the invention remaining the same, the details of construction and forms of embodiment may be varied widely with respect to those described and illustrated, without thereby departing from the scope of the present invention as defined in the appended claims.

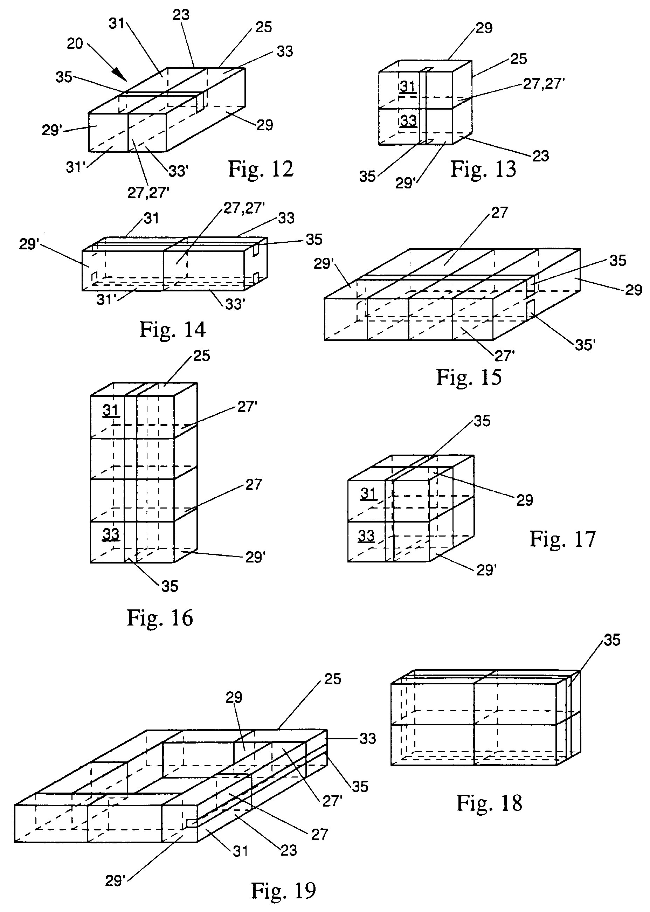

Figures 12, 13 and 14 show a package according to the invention consisting of two polyethylene diaper bags 23,25. In the package of Figures 12 and 13, two longitudinal side faces 27,27' of the bags 23,25 are contiguous. The bags are held in a fixed position by adhesive tape 35, which is sufficiently strong to enable the bags 25 and 23 to be carried, or otherwise transported, as a single unit. The tape 35 extends from the lateral side face 29 of bag 25, across the transverse side faces 31 and 33 of both bags 25 and 23 to the lateral side face 29' of bag 23. In this way, relative movement of bags 23 and 25 is prevented, both in a direction parallel to the plane of side faces 29,29' as well as in a direction perpendicular to the plane of the side faces 29,29'.

In the package according to Figure 14, the bags 23,25 are aligned in their longitudinal direction. Two tapes 35,35' provide for a stable connection of the bags that can be easily undone.

In the package according to Figures 15 to 18, a plurality of bags is connected to form a single package by means of tape 35. The tape 35 can comprise a number of separate tape members, or can consist of one or more single strips that completely encircle the package. In the package according to Figure 19, a multiplicity of bags 25, 23 is configured and connected for shipment on a pallet of predetermined dimensions.

## Claims

1. A package comprising a plurality of packs (2) placed side by side and fixed together by means of one or more pieces of adhesive tape (7) so that they can be separated one at a time, leaving the rest of said package intact, characterised in that
each of said packs (2) comprises disposable articles and a wrapper of flexible material, said adhesive tape (7) being adhesively attached to each of said packs (2) and being detachable from each of said packs (2) without tearing said flexible material.

2. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces, said shorter sides (5) and two outer longer sides (6) of two end packs face outwardly, wherein said packs are bound together by a single piece of adhesive tape wound all the way around said plurality of packs on the outwardly facing side (5), (6) thereof.

3. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces, said shorter sides (5) and two outer longer sides (6) of the two end packs face outwardly, wherein said packs are bound together by a single piece of adhesive tape at each end, fixed transversely at least to said adjacent shorter sides (5).

4. A package according to claim 3 wherein said pieces of adhesive tape (7) extend with their ends at least partially on said two longer sides of said package.

5. A package according to claims 2 to 4 wherein said piece of adhesive tape (7) is positioned substantially at the centres of said adjacent shorter sides (5), parallel to the upper and lower edges thereof.

6. A package according to claims 2 and 5 wherein said adhesive tape (7) covers, in total, at least 20 percent and preferably at least 40 percent of the total surface area of said shorter sides (5) of said plurality of packs.

7. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces, said shorter sides (5) and two outer longer sides (6) of the two end packs face outwardly, wherein said packs are bound together by at least two pieces of said adhesive tape (7) wound all the way around the outwardly facing sides (5, 6) of said plurality of packs.

8. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces, said shorter sides (5) and two outer longer sides (6) of the two end packs face outwardly, wherein said packs are bound together by at least two pieces of said adhesive tape (7) fixed transversely at least to said adjacent short sides (5) at each end.

9. A package according to claim 8 wherein said at least two pieces of adhesive tape (7) at each end extend with their ends at least partially on said longer sides (6) of said package.

10. A package according to claims 7 to 9 wherein said at least two pieces of adhesive tape (7) on said adjacent shorter sides (5) at each end are parallel to each other and to upper and lower edges of said shorter sides (5) with at least one piece of said adhesive tape positioned within upper halves of said shorter sides (5) and at least one piece of said adhesive tape positioned within lower halves of said shorter sides (5).

11. A package according to claim 7 to 9 wherein said pieces of adhesive tape (7) cover, in total, at least 20 percent of total surface area of said shorter sides (5) of said plurality of packs.

12. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces, said shorter sides (5) and two outer longer sides (6) of the two end packs face outwardly, wherein said packs are bound together by at least two pieces of said adhesive tape (7) wound all he way around said upper and lower faces and said two longer sides (6) of said plurality of packs.

13. A package comprising a plurality of packs according to claim 1, each of said packs comprising two longer sides, two shorter sides (5), an upper face and a lower face, said packs being disposed side by side in a single row with their longer sides next to each other in a manner such that said upper and lower faces of said packs, said shorter sides (5) of said packs and two outer longer sides (6) of end packs face outwardly, wherein said packs are bound together by at least two pieces of said adhesive tape (7) fixed transversely on at least said adjacent upper and lower faces at each end.

14. A package according to claim 13 wherein said pieces of adhesive tape (7) extend with their ends at least partially on said longer sides of said package.

15. A package according to claims 12 to 14 wherein each of said at least two pieces of adhesive tape (7) on said upper and lower faces respectively is positioned within the third of the surface of each said face which is adjacent said shorter side (5).

16. A package according to claims 12 to 15 wherein said pieces of adhesive tape (7) cover at least 20 percent of the total surface area of said upper and lower faces of said plurality of packs.

17. A package according to any of the preceding claims wherein at least one end of the or each said at least one piece of adhesive tape (7) has an adhesive-free region (8), which can be gripped in order to remove said tape.

18. A package according to any of the preceding claims wherein at least one end of the or each said at least one piece of adhesive tape (7) has a region in which the adhesive has been neutralised, which can be gripped in order to remove said tape.

19. A package according to any of the preceding claims wherein said packs comprise disposable nappies.

20. A package according to any of the preceding claims wherein said the or said each at least one piece of adhesive tape (7) has a peel strength of between 0.2 N/cm and 2.5 N/cm, preferably between 0.8 N/cm and 1.6 N/cm, and a shear strength of at least 500 minutes at ambient temperature and at least 50-60 minutes at 50 degrees celsius in relation to said outer surfaces of said packs (2) to which it is fixed.

21. A package according to any of the preceding claims wherein said the or said at least one piece of adhesive tape (7) is made of transparent material.

22. A package according to any of the preceding claims wherein a bar code (9) and an alphanumeric code (10) for identifying products contained are applied to said the or said at least one piece of adhesive tape (7) and comprise an opaque region (11) corresponding at least to said bar code (9) on said the or said each piece (7).

23. A package according to any of the preceding claims wherein at least one of said packs comprises a handle, each said at least one handle being capable of supporting weight of entire said package.

## Patentansprüche

1. Eine Verpackung, welche eine Mehrzahl von Packungen (2) umfaßt, welche Seite an Seite angeordnet und mittels eines Stücks oder mehrerer Stücke von Klebeband (7) zusammen fixiert sind, sodaß sie jeweils einzeln getrennt werden können, wobei der Rest der genannten Verpackung intakt gelassen wird, dadurch gekennzeichnet,
daß jede der genannten Packungen (2) wegwerfbare Artikel und eine Hülle aus flexiblem Material umfaßt, wobei das genannte Klebeband (7) mit jeder der genannten Packungen (2) klebend verbunden ist und von jeder der genannten Packungen (2) ohne Reißen des genannten flexiblen Materials lösbar ist.

2. Eine Verpackung, welche eine Mehrzahl von Packungen umfaßt, nach Anspruch 1, bei welcherjede der genannten Packungen zwei längere Seiten, zwei kürzere Seiten (5), eine Oberseite und eine Unterseite umfaßt, wobei die genannten Packungen Seite an Seite in einer einzigen Reihe mit ihren längeren Seiten am nächsten zueinander derartig angeordnet sind, daß die genannten oberen und unteren Seiten, die genannten kürzeren Seiten (5) und zwei äußere längere Seiten (6) zweier Endpackungen nach auswärts gewandt sind, wobei die genannten Packungen durch ein einziges Stück Klebeband, welches den gesamten Weg rund um die genannte Vielzahl von Packungen an deren auswärts gewandter Seite (5,6) gewunden ist, zusammen gebunden sind.

3. Eine Verpackung, welche eine Mehrzahl von Packungen umfaßt, nach Anspruch 1, bei welcher jede der genannten Packungen zwei längere Seiten, zwei kürzere Seiten (5), eine Oberseite und eine Unterseite umfaßt, wobei die genannten Packungen Seite an Seite in einer einzigen Reihe mit ihren längeren Seiten am nächsten zueinander derartig angeordnet sind, daß die genannten oberen und unteren Seiten, die genannten kürzeren Seiten (5) und zwei äußere längere Seiten (6) der zwei Endpackungen auswärts gewandt sind, wobei die genannten Packungen an jedem Ende durch ein einziges Stück Klebeband, welches mindestens an den genannten anliegenden kürzeren Seiten (5) querlaufend fixiert ist, zusammen gebunden sind.

4. Eine Verpackung nach Anspruch 3, bei welcher die genannten Stücke Klebeband (7) sich mit ihren Enden mindestens teilweise an den genannten zwei längeren Seiten der genannten Verpackung erstrecken.

5. Eine Verpackung nach den Ansprüchen 2 bis 4, bei welcher das genannte Stück Klebeband (7) im wesentlichen an den Mitten der genannten anliegenden kürzeren Seiten (5), parallel zu deren oberen und unteren Rändern, positioniert ist.

6. Eine Verpackung nach den Ansprüchen 2 und 5, bei welcher das genannte Klebeband (7), insgesamt, mindestens 20 % und vorzugsweise mindestens 40 % der gesamten Oberfläche der genannten kürzeren Seiten (5) der genannten Mehrzahl von Packungen bedeckt.

7. Eine Verpackung, welche eine Mehrzahl von Packungen umfaßt, nach Anspruch 1, bei welcher jede der genannten Packungen zwei längere Seiten, zwei kürzere Seiten (5), eine obere Seite und eine untere Seite umfaßt, wobei die genannten Packungen Seite an Seite in einer einzigen Reihe mit ihren längeren Seiten am nächsten zueinander derartig angeordnet sind, daß die genannten oberen und unteren Seiten, die genannten kürzeren Seiten (5) und zwei äußere längere Seiten (6) der zwei Endpackungen auswärts gewandt sind, bei welcher die genannten Packungen durch mindestens zwei Stücke des genannten Klebebandes (7), welches den ganzen Weg rund um die auswärts gewandten Seiten (5, 6) der genannten Mehrzahl von Packungen gewunden ist, zusammengebunden sind.

8. Eine Verpackung, welche eine Mehrzahl von Packungen umfaßt, nach Anspruch 1, wobei jede der genannten Packungen zwei längere Seiten, zwei kürzere Seiten (5), eine obere Seite und eine untere Seite umfaßt, wobei die genannten Packungen Seite an Seite in einer einzigen Reihe mit ihren längeren Seiten am nächsten zueinander derartig angeordnet sind, daß die genannten oberen und unteren Seiten, die genannten kürzeren Seiten (5) und zwei äußere längere Seiten (6) der zwei Endpackungen auswärts gewandt sind, bei welcher die genannten Packungen durch mindestens zwei Stücke des genannten Klebebandes (7), welches mindestens an den genannten anliegenden kurzen Seiten (5) an jedem Ende querlaufend fixiert ist, zusammen gebunden sind.

9. Eine Verpackung nach Anspruch 8, bei welcher die genannten mindestens zwei Stücke Klebeband (7) an jedem Ende sich mit ihren Enden mindestens teilweise an den genannten längeren Seiten (6) der genannten Verpackung erstrecken.

10. Eine Verpackung nach den Ansprüchen 7 bis 9, bei welcher die genannten mindestens zwei Stücke Klebeband (7) an den genannten benachbaden kürzeren Seiten (5) an jedem Ende parallel zueinander und zu den oberen und unteren Rändern der genannten kürzeren Seiten (5) sind, wobei mindestens ein Stück des genannten Klebebandes innerhalb oberer Hälften der genannten kürzeren Seiten (5) positioniert ist und mindestens ein Stück des genannten Klebebandes innerhalb unterer Hälften der genannten kürzeren Seiten (5) positioniert ist.

11. Eine Verpackung nach Anspruch 7 bis 9, bei welcher die genannten Stücke Klebeband (7), insgesamt, mindestens 20 % der gesamten Oberfläche der genannten kürzeren Seiten (5) der genannten Mehrzahl von Packungen bedecken.

12. Eine Verpackung, welche eine Mehrzahl von Packungen umfaßt, nach Anspruch 1, wobei jede der genannten Packungen zwei längere Seiten, zwei kürzere Seiten (5), eine obere Seite und eine untere Seite umfaßt, wobei die genannten Packungen Seite an Seite in einer einzigen Reihe mit ihren längeren Seiten am nächsten zueinander derart angeordnet sind, daß die genannten oberen und unteren Seiten, die genannten kürzeren Seiten (5) und zwei äußere längere Seiten (6) der zwei Endpackungen auswärts gewandt sind, bei welcher die genannten Packungen durch mindestens zwei Stücke des genannten Klebebandes (7), welches den ganzen Weg rund um die genannten oberen und unteren Seiten und die genannten zwei längeren Seiten (6) der genannten Mehrzahl von Packungen gewunden ist, zusammen gebunden sind.

13. Eine Verpackung, welche eine Mehrzahl von Packungen enthält, nach Anspruch 1, wobei jede der genannten Packungen zwei längere Seiten, zwei kürzere Seiten (5), eine obere Seite und eine untere Seite umfaßt, die genannten Packungen Seite an Seite in einer einzigen Reihe mit ihren längeren Seiten am nächsten zueinander auf eine Art angeordnet sind, daß die genannten oberen und unteren Seiten der genannten Packungen, die genannten kürzeren Seiten (5) der genannten Packungen und zwei äußere längere Seiten (6) von Endpackungen auswärts gewandt sind, bei welcher die genannten Packungen durch mindestens zwei Stücke des genannten Klebebandes (7), welches an mindestens den genannten anliegenden oberen und unteren Seiten an jedem Ende querlaufend fixiert ist, zusammengebunden sind.

14. Eine Verpackung nach Anspruch 13, bei welcher die genannten Stücke Klebeband (7) sich mit ihren Enden mindestens teilweise an den genannten längeren Seiten der genannten Verpackung erstrecken.

15. Eine Verpackung nach den Ansprüchen 12 bis 14, bei welcher jedes der genannten mindestens zwei Stücke Klebeband (7) an den genannten oberen und unteren Seiten jeweils innerhalb des Drittels der Oberfläche jeder genannten Seite, welches der genannten kürzeren Seite (5) anliegend ist, positioniert ist.

16. Eine Verpackung nach den Ansprüchen 12 bis 15, bei welcher die genannten Stücke Klebeband (7) mindestens 20 % der gesamten Oberfläche der genannten oberen und unteren Flächen der genannten Mehrzahl von Packungen bedecken.

17. Eine Verpackung nach einem der vorhergehenden Ansprüche, bei welcher mindestens ein Ende eines jeden genannten mindestens einen Stücks Klebeband (7) einen Klebstoff-freien Bereich (8) umfaßt, welcher ergriffen werden kann, um das genannte Band zu entfernen.

18. Eine Verpackung nach einem der vorhergehenden Ansprüche, bei welcher mindestens ein Ende jedes genannten mindestens einen Stücks Klebeband (7) einen Bereich aufweist, in welchem der Klebstoff neutralisiert worden ist, welcher ergriffen werden kann, um das genannte Band zu entfernen.

19. Eine Verpackung nach einem der vorhergehenden Ansprüche, bei welcher die genannten Packungen Wegwerfwindeln umfassen.

20. Eine Verpackung nach einem der vorhergehenden Ansprüche, bei welcher jedes genannte mindestens eine Stück Klebeband (7) eine Abziehfestigkeit von zwischen 0,2 N/cm und 2,5 N/cm, vorzugsweise zwischen 0,8 N/cm und 1,6 N/cm, aufweist und eine Scherfestigkeit von mindestens 500 Minuten bei Umgebungstemperatur und mindestens 50 bis 60 Minuten bei 50 °C im Verhältnis zu den genannten äußeren Oberflächen der genannten Packungen (2), an welchen es fixiert ist, aufweist.

21. Eine Verpackung nach einem der vorhergehenden Ansprüche, bei welcher das genannte mindestens eine Stück Klebeband (7) aus transparentem Material hergestellt ist.

22. Eine Verpackung nach einem der vorhergehenden Ansprüche, bei welcher ein Strichcode (9) und ein alphanumerischer Code (10) zum Identifizieren von enthaltenen Produkten auf das genannte mindestens eine Stück Klebeband (7) aufgebracht sind und einen lichtundurchlässigen Bereich (11) umfassen, welcher mindestens dem genannten Strichcode (9) an dem genannten jeden Stück Klebeband (7) entspricht.

23. Eine Verpackung nach einem der vorhergehenden Ansprüche, bei welcher mindestens eine der genannten Packungen einen Griff umfaßt, wobei jeder genannte mindestens eine Griff imstande ist, das Gewicht der gesamten genannten Verpackung zu tragen.

## Revendications

1. Emballage comportant une pluralité de paquets (2) disposés côte à côte et fixés ensemble par l'intermédiaire d'une ou plusieurs pièces de ruban adhésif (7) de telle sorte qu'ils peuvent être séparés un par un en laissant le reste dudit emballage intact, caractérisé en ce que :
chacun desdits paquets (2) comprend des articles à jeter après usage et une enveloppe en matériau souple, ledit ruban adhésif (7) étant fixé de façon adhésive à chacun desdits paquets (2) et étant susceptible d'être détaché de chacun desdits paquets (2) sans déchirer ledit matériau souple.

2. Emballage comportant une pluralité de paquets selon la revendication 1, chacun desdits paquets comprenant deux côtés plus longs, deux côtés plus courts (5), une face supérieure et une face inférieure, lesdits paquets étant disposés côte à côte en une seule rangée, leurs côtés plus longs étant proches les uns des autres si bien que lesdites faces supérieure et inférieure, lesdits côtés plus courts (5) et deux côtés extérieurs plus longs (6) de deux paquets d'extrémité sont tournés vers l'extérieur, dans lequel lesdits paquets sont reliés entre eux par une seule pièce d'un ruban adhésif enroulé tout autour de ladite pluralité de paquets sur le côté tourné vers l'extérieur (5), (6) de ceux-ci.

3. Emballage comportant une pluralité de paquets selon la revendication 1, chacun desdits paquets comprenant deux côtés plus longs, deux côtés plus courts (5), une face supérieure et une face inférieure, lesdits paquets étant disposés côte à côte en une seule rangée, leurs côtés plus longs étant proches les uns des autres si bien que lesdites faces supérieure et inférieure, lesdits côtés plus courts (5) et les deux côtés extérieurs plus longs (6), de deux paquets d'extrémité sont tournés vers l'extérieur, dans lequel lesdits paquets sont reliés entre eux par une seule pièce d'un ruban adhésif à chaque extrémité, fixé transversalement au moins auxdits côtés adjacents plus courts (5).

4. Emballage selon la revendication 3, dans lequel lesdites pièces de ruban adhésif (7) s'étendent, avec leurs extrémités, au moins partiellement, sur lesdits deux côtés plus longs dudit emballage.

5. Emballage selon les revendications 2 à 4, dans lequel ladite pièce de ruban adhésif (7) est disposée sensiblement aux centres desdits côtés adjacents plus courts (5) parallèlement aux bords supérieur et inférieur de ceux-ci.

6. Emballage selon les revendications 2 et 5, dans lequel ledit ruban adhésif (7) recouvre, en totalité, au moins 20% et, de préférence, au moins 40%, de la surface totale desdits côtés plus courts (5) de ladite pluralité de paquets.

7. Emballage comportant une pluralité de paquets selon la revendication 1, chacun desdits paquets comprenant deux côtés plus longs, deux côtés pins courts (5), une face supérieure et une face inférieure, lesdits paquets étant disposés côte à côte en une seule rangée, leurs côtés plus longs étant proches les uns des autres si bien que lesdites faces supérieure et inférieure, lesdits côtés plus courts (5) et deux côtés extérieurs plus longs (6) de deux paquets d'extrémité sont tournés vers l'extérieur, dans lequel lesdits paquets sont reliés entre eux par au moins deux pièces dudit ruban adhésif (7) enroulé tout autour des côtés tournés vers l'extérieur (5, 6) de ladite pluralité de paquets.

8. Emballage comportant une pluralité de paquets selon la revendication 1, chacun desdits paquets comprenant deux côtés plus longs, deux côtés plus courts (5), une face supérieure et une face inférieure, lesdits paquets étant disposés côte à côte en une seule rangée, leurs côtés plus longs étant proches les uns des autres si bien que lesdites faces supérieure et inférieure, lesdits côtés plus courts (5) et deux côtés extérieurs plus longs (6) de deux paquets d'extrémité sont tournés vers l'extérieur, dans lequel lesdits paquets sont reliés entre eux par au moins deux pièces dudit ruban adhésif (7) fixé transversalement au moins auxdits côtés adjacents courts (5), à chaque extrémité.

9. Emballage selon la revendication 8, dans lequel au moins lesdites deux pièces de ruban adhésif (7) à chaque extrémité, s'étendent avec leurs extrémités, au moins partiellement, sur lesdits côtés plus longs (6) dudit emballage.

10. Emballage selon les revendications 7 à 9, dans lequel au moins lesdites deux pièces de ruban adhésif (7), sur lesdits côtés adjacents plus courts (5), à chaque extrémité, sont parallèles entre elles et aux bords supérieur et inférieur desdits côtés plus courts (5), au moins une pièce dudit ruban adhésif étant disposée dans les moitiés supérieures desdits côtés plus couds (5) et au moins une pièce dudit ruban adhésif étant disposée dans les moitiés inférieures desdits côtés plus couds (5).

11. Emballage selon les revendications 7 à 9, dans lequel lesdites pièces de ruban adhésif (7) recouvrent, en totalité, au moins 20% de la surface totale desdits côtés plus couds (5) de ladite pluralité de paquets.

12. Emballage comportant une pluralité de paquets selon la revendication 1, chacun desdits paquets comprenant deux côtés plus longs, deux côtés plus couds (5), une face supérieure et une face inférieure, lesdits paquets étant disposés côte à côte en une seule rangée, leurs côtés plus longs étant proches les uns des autres si bien que lesdites faces supérieure et inférieure, lesdits côtés plus couds (5) et deux côtés extérieurs plus longs (6) de deux paquets d'extrémité sont tournés vers l'extérieur, dans lequel lesdits paquets sont reliés entre eux par au moins deux pièces dudit ruban adhésif (7) enroulé tout autour desdites faces supérieure et inférieure et desdits deux côtés plus longs (6) de ladite pluralité de paquets.

13. Emballage comportant une pluralité de paquets selon la revendication 1, chacun desdits paquets comprenant deux côtés plus longs, deux côtés plus couds (5), une face supérieure et une face inférieure, lesdits paquets étant disposés côte à côte en une seule rangée, leurs côtés plus longs étant proches les uns des autres si bien que lesdites faces supérieure et inférieure desdits paquets, lesdits côtés plus couds (5) desdits paquets et deux côtés extérieurs plus longs (6) des paquets d'extrémité sont tournés vers l'extérieur, dans lequel lesdits paquets sont reliés entre eux par au moins deux pièces dudit ruban adhésif (7) fixé transversalement sur au moins lesdites faces adjacentes supérieure et inférieure, à chaque extrémité.

14. Emballage selon la revendication 13, dans lequel lesdites pièces de ruban adhésif (7) s'étendent avec leurs extrémités, au moins partiellement, sur lesdits côtés plus longs dudit emballage.

15. Emballage selon les revendications 12 à 14, dans lequel chacune au moins desdites deux pièces de ruban adhésif (7) sur lesdites faces supérieure et inférieure est disposée respectivement dans le tiers de la surface de chaque dite face qui est adjacente audit côté plus court (5).

16. Emballage selon les revendications 12 à 15, dans lequel lesdites pièces de ruban adhésif (7) recouvrent au moins 20% de la surface totale desdites faces supérieure et inférieure de ladite pluralité de paquets.

17. Emballage selon l'une quelconque des revendications précédentes, dans lequel au moins une extrémité, ou chacune desdites au moins une pièce de ruban adhésif (7), comporte une zone exempte d'adhésif (8), qui peut être saisie afin d'enlever ledit ruban.

18. Emballage selon l'une quelconque des revendications précédentes, dans lequel au moins une extrémité, ou chacune desdites au moins une pièce de ruban adhésif (7), comporte une zone dans laquelle l'adhésif a été neutralisé, qui peut être saisie afin d'enlever ledit ruban.

19. Emballage selon l'une quelconque des revendications précédentes, dans lequel lesdits paquets renferment des serviettes à jeter après usage.

20. Emballage selon l'une quelconque des revendications précédentes, dans lequel ladite ou chacune au moins une pièce de ruban adhésif (7) a une résistance à l'arrachement située entre 0,2N/cm et 2,5 N/cm, de préférence entre 0,8N/cm et 1,6 N/cm, et une résistance au cisaillement d'au moins 500 minutes à la température ambiante et d'au moins 50-60 minutes, à 50°Celsius, par rapport à ladite surface extérieure desdits paquets (2) à laquelle elle est fixée.

21. Emballage selon l'une quelconque des revendications précédentes, dans lequel ladite ou lesdites au moins une pièce de ruban adhésif (7) est réalisée en un matériau transparent.

22. Emballage selon l'une quelconque des revendications précédentes, dans lequel un code barre (9) et un code alpha numérique (10) sont prévus pour identifier les articles contenus et sont appliqués auxdites ou à ladite au moins une pièce de ruban adhésif (7) et comprennent une zone opaque (11) correspondant au moins audit code baffe (9) disposé sur ladite ou sur chacune desdites pièces (7).

23. Emballage selon l'une quelconque des revendications précédentes, dans lequel au moins l'un desdits paquets comporte une poignée, au moins chaque dite poignée étant susceptible de supporter le poids dudit emballage tout entier.
